(19) Europäisches Patentamt
European Patent Office
Office européen des brevets

(11) Veröffentlichungsnummer: **0 320 677 B1**

(12) **EUROPÄISCHE PATENTSCHRIFT**

(45) Veröffentlichungstag der Patentschrift: **03.02.93**

(51) Int. Cl.$^5$: **C07D 409/10**, C07D 411/10, A01N 43/38

(21) Anmeldenummer: **88119635.6**

(22) Anmeldetag: **25.11.88**

Die Akte enthält technische Angaben, die nach dem Eingang der Anmeldung eingereicht wurden und die nicht in dieser Patentschrift enthalten sind.

# (54) N-Phenyltetrahydrophthalimidverbindungen.

(30) Priorität: **05.12.87 DE 3741273**

(43) Veröffentlichungstag der Anmeldung:
**21.06.89 Patentblatt 89/25**

(45) Bekanntmachung des Hinweises auf die Patenterteilung:
**03.02.93 Patentblatt 93/05**

(84) Benannte Vertragsstaaten:
**BE CH DE ES FR GB IT LI NL SE**

(56) Entgegenhaltungen:
**EP-A- 0 207 894**
**EP-A- 0 289 910**
**US-A- 3 772 334**
**ZA-A- 8 703 933**

(73) Patentinhaber: **BASF Aktiengesellschaft**
**Carl-Bosch-Strasse 38**
**W-6700 Ludwigshafen(DE)**

(72) Erfinder: **Rueb, Lothar, Dr.**
**Zum Weidentor 4**
**W-6720 Speyer(DE)**
Erfinder: **Eicken, Karl, Dr.**
**Am Huettenwingert 12**
**W-6706 Wachenheim(DE)**
Erfinder: **Schwalge, Barbara, Dr.**
**Adolf-Diesterweg-Strasse 77**
**W-6700 Ludwigshafen(DE)**
Erfinder: **Plath, Peter, Dr.**
**Hans-Balcke-Strasse 13**
**W-6710 Frankenthal(DE)**
Erfinder: **Wuerzer, Bruno, Dr.**
**Ruedigerstrasse 13**
**W-6701 Otterstadt(DE)**
Erfinder: **Meyer, Norbert, Dr.**
**Dossenheimer Weg 22**
**W-6802 Ladenburg(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es sind bereits N-arylsubstituierte Tetrahydrophthalimide mit Herbizidwirkung bekannt, z.B. werden in EP 207 894 u.a. Tetrahydrophthalimide der Formel I' beschrieben,

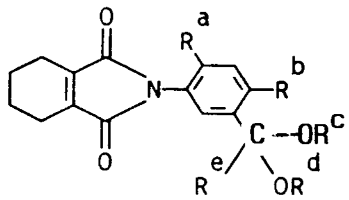

I'

in der die Reste beispielsweise folgende Bedeutung haben:

- $R^a$ und $R^b$ unabhängig voneinander Wasserstoff oder Halogenatom
- $R^c$ und $R^d$ unter anderem gemeinsam eine gegebenenfalls substituierte $C_2$-$C_3$-Alkylenbrücke und
- $R^e$ unter anderem Wasserstoff, Cyanogruppe oder $C_1$-$C_4$-Alkylgruppe.

Diese bekannten Verbindungen zeigen jedoch bei geringen Aufwandmengen eine unbefriedigende Wirksamkeit.

Außerdem sind der EP-A 289 910 u.a. herbizid wirksame substituierte Tetrahydrophthalimide der Formel I''

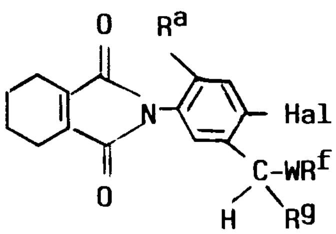

I''

zu entnehmen, wobei die Variablen folgende Bedeutung haben:

- $R^a$ Wasserstoff oder Halogen;
- Hal Halogen;
- W Sauerstoff oder Schwefel;
- $R^f$ Wasserstoff, $C_1$-$C_4$-Alkyl, das durch Cycloalkyl, Halogen, Alkoxy, Alkylthio, Dialkylamino, Cyano, Nitro, Phenyl, Trialkylsilyl, Benzyloxy oder Heterocyclyl substituiert sein kann;
- $R^g$ Hydroxyl, $C_1$-$C_4$-Alkoxy, $C_1$-$C_4$-Alkylthio oder
- $WR^f$ und $R^g$ zusammen eine Gruppe $-W-CH_2-CH_2-W-$.

Der Erfindung lag daher die Aufgabe zugrunde, N-Phenyltetrahydrophthalimidverbindungen zu finden, die bei geringer Aufwandmenge eine bessere Wirksamkeit gegen unerwünschte Pflanzen zeigen, ohne die Kulturpflanzen zu schädigen.

Entsprechend dieser Aufgabe wurde gefunden, daß Tetrahydrophthalimide der allgemeinen Formel I

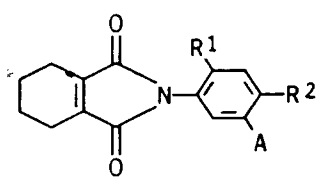

I

in der die Substituenten die folgende Bedeutung haben:

- $R^1$ Wasserstoff oder Halogen
- $R^2$ Halogen
- A einen Substituenten der Formel

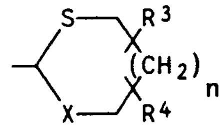

oder

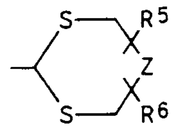

wobei die Variablen die folgende Bedeutung haben:

- X Sauerstoff oder Schwefel;
- n null oder eins;
- $R^3$ Wasserstoff, die Hydroxylgruppe, die Carboxylgruppe, eine $C_1$-$C_6$-Alkoxycarbonylgruppe, eine $C_1$-$C_3$-Alkylgruppe, die durch Halogen, Cyano, Hydroxy, Mercapto, $C_1$-$C_6$-Alkoxycarbonyl, $C_1$-$C_4$-Alkoxy, $C_1$-$C_4$-Alkylthio, $C_3$-$C_6$-Alkenyloxy, $C_3$-$C_6$-Alkenylthio, $C_3$-$C_6$-Alkinyloxy, $C_3$-$C_6$-Alkinylthio, oder $C_1$-$C_4$-Acyloxy substituiert sein kann, oder eine im Alkylether- oder Thioetherteil durch $C_1$-$C_6$-Alkoxycarbonyl substituierte $C_1$-$C_4$-Alkoxy-$C_1$-$C_2$-alkyl- oder $C_1$-$C_4$-Alkylthio-$C_1$-$C_2$-alkylgruppe;
- $R^4$ Wasserstoff oder $C_1$-$C_3$-Alkyl;
- Z Methylenoxymethylen, Methylenthiomethylen oder Ethenylen (-CH = CH-);
- $R^5$ Wasserstoff oder $C_1$-$C_3$-Alkyl;
- $R^6$ Wasserstoff oder $C_1$-$C_3$-Alkyl;

unabhängig von der jeweiligen sterischen Anordnung, ausgenommen Verbindungen I, in denen A einen Substituenten der Formel

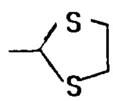

bedeutet, es sei denn $R^1$ steht für Wasserstoff und $R^2$ für Chlor,
eine vorteilhafte herbizide Wirkung, besonders im Nachauflaufverfahren, haben und gegenüber einer Reihe von Kulturpflanzen selektiv sind.

Unter Halogen wie vorstehend verwendet, ist Fluor, Chlor oder Brom zu verstehen, wobei $R^1$ in der Bedeutung Halogen bevorzugt für Fluor und $R^2$ bevorzugt für Chlor steht.

Die Bezeichnung Alkyl (allein oder als Teil eines Substituenten) umfaßt verzweigte und geradkettige Reste, z.B. Methyl, Ethyl, n-Propyl und Isopropyl.

Die Alkenyl- und Alkinylreste können ebenfalls verzweigt oder geradkettig sein. Beispiele für Alkenylreste in Formel I sind Allyl, 2-Butenyl, 3-Butenyl, 2-Isobutenyl, 2-Pentenyl, 3-Pentenyl und 4-Pentenyl, insbesondere Allyl oder 2-Butenyl, Alkinylreste sind in der Regel Propargyl, 2-Butinyl, 3-Butinyl sowie isomere Pentinylreste; vorzugsweise ist der Alkinylrest jedoch ein Propargyl- oder 2- oder 3-Butinylrest.

Als Substituent der allgemeinen Formel I bedeutet Alkoxy: Methoxy, Ethoxy, n-Propyloxy, i-Propyloxy sowie die vier isomeren Butyloxyreste, insbesondere Methoxy, Ethoxy oder i-Propyloxy. Beispiele für Alkylthio in der Formel I sind n-Propylthio, i-Propylthio und n-Butylthio sowie insbesondere Methylthio und Ethylthio.

Bei Substitution im Thioacetalteil umfaßt die Formel I sowohl Enantiomere, Diastereomere als auch deren Gemische.

Die Verbindung der Formel I können als N-substituierte Tetrahydrophthalimide aufgefaßt werden; sie sind demnach aus 3.4.5.6-Tetrahydrophthalsäureanhydrid und einem Anilin der Formel V z.B. in einem Lösungsmittel bei einer Temperatur von 0 °C bis 150 °C, vorzugsweise 20 °C bis 100 °C, erhältlich. Als Lösungsmittel eignen sich z.B. niedere Alkansäuren wie Eisessig und Propionsäure und aprotische Lösungsmittel wie Toluol und Xylol in Gegenwart von sauren Katalysatoren wie aromat. Sulfonsäuren.

Unter den Verbindungen I sind diejenigen bevorzugt, in denen $R^1$ Wasserstoff oder Fluor, $R^2$ Chlor und

A Dithian, Dithiolan, Oxathiolan oder deren substituierte Analoge bedeutet.

Die Aniline der Formel V können z.B. dadurch erhalten werden, daß man ein entsprechend substituiertes Nitrobenzol IV durch Reduktionsmittel wie Eisen oder ein Zinn(II)-Salz reduziert.

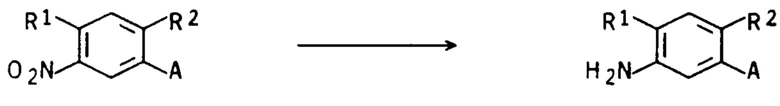

IV V

Die Reduktion kann auch als katalytische Hydrierung an einem Metallkatalysator wie Platin, Palladium und Raney-Nickel unter relativ milden Bedingungen durchgeführt werden.

Die Nitrobenzole IV sind durch Umsetzung der Benzaldehyde II in einem Lösungsmittel in Gegenwart einer Säure mit einem Dithiol oder Mercaptoalkanol der Formel IIIa bzw. IIIb zugänglich.

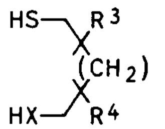 IIIa oder 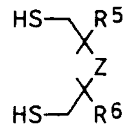 IIIb

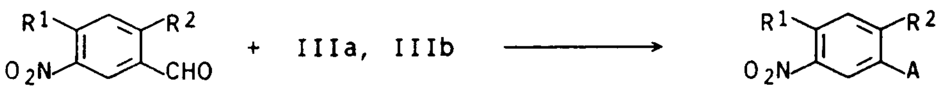

\+ IIIa, IIIb

II IV

Die in den nachstehenden Beispielen wiedergegebenen Arbeitsempfehlungen wurden unter entsprechender Abwandlung der Ausgangsverbindungen zur Gewinnung weiterer Verbindungen der allgemeinen Formel I benutzt. Die Verbindungen sind mit physikalischen Daten in den folgenden Tabellen aufgeführt. Verbindungen ohne solche Angaben lassen sich aus entsprechenden Stoffen in analoger Weise erhalten. Sie lassen aufgrund ihrer nahen strukturellen Beziehung zu den hergestellten und untersuchten Verbindungen eine gleichartige Wirkung erwarten.

Beispiel

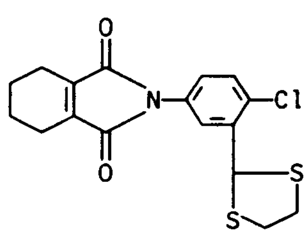

a) Zu 18,6 g 2-Chlor-5-nitro-benzaldehyd und 0,5 g p-Toluolsulfonsäure in 250 ml Toluol werden 9,9 g Ethan-1,2-dithiol gegeben und 5 Stunden unter Rückfluß am Wasserabscheider gekocht. Nach dem Abkühlen wird das Lösungsmittel entfernt, der Rückstand mit Petrolether verrührt, abfiltriert und getrocknet. Man erhält 25,5 g 4-Chlor-3-(1.3-dithiolan-2-yl)-nitrobenzol (Fp. 130 - 131 ° C).

b) Zu einer Mischung von 15 ,9 g Eisenpulver in 50 ml Methanol und 75 ml Eisessig gibt man bei Rückfluß portionsweise 24,9 g der obigen Nitroverbindung und erhitzt 2 Stunden am Rückfluß. Nach dem Abkühlen werden 250 ml Wasser zugegeben und der Feststoff abfiltriert. Das Filtrat wird 3 mal mit je 100 ml Essigester extrahiert, getrocknet, eingeengt, aus Petrolether ausgefällt, abgesaugt und getrocknet. Man erhält 21,5 g 4-Chlor-3-(1.3-dithiolan-2-yl)-anilin (Fp. 60 - 63 ° C).

c) 11,6 g des obigen Anilins und 7,6 g Cyclohexen-1,2-dicarbonsäureanhydrid werden in 150 ml Eisessig 2 Tage bei Raumtemperatur gerührt, der ausgefallene Niederschlag abfiltriert, mit Wasser und Petrolether gewaschen und getrocknet. Man erhält 13 g N-[4-chlorphenyl-3-(1.3-Dithiolan-2-yl)]-3.4.5.6-tetrahydro-phthalimid (Fp. 155 - 158° C) (Tab. 1 Nr. 1.001)

Weitere Beispiele für Wirkstoffe, die nach diesem Syntheseprinzip hergestellt werden können, finden sich in den Tabellen 1 bis 5.

Tabelle 1

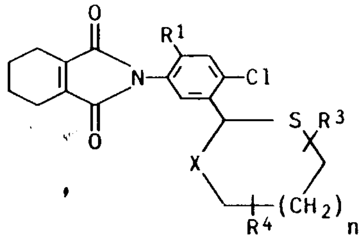

| Nr. | $R^1$ | $R^3$ | $R^4$ | n | X | Fp. [°C] |
|---|---|---|---|---|---|---|
| 1.001 | H | H | H | 0 | S | 155-158 |
| 1.002 | H | 4-$CH_3$ | H | 0 | S | 101-102 |
| 1.003 | F | 4-$CH_3$ | H | 0 | S | 138-139 |
| 1.004 | H | 4-$CH_2CH_3$ | H | 0 | S | |
| 1.005 | F | 4-$CH_2CH_3$ | H | 0 | S | |
| 1.006 | H | 4-$(CH_2)_2CH_3$ | H | 0 | S | |
| 1.007 | F | 4-$(CH_2)_2CH_3$ | H | 0 | S | |
| 1.008 | H | 4-$CH(CH_3)_2$ | H | 0 | S | |
| 1.009 | F | 4-$CH(CH_3)_2$ | H | 0 | S | |
| 1.010 | H | 4-$CH_2Cl$ | H | 0 | S | 109-110 |
| 1.011 | F | 4-$CH_2Cl$ | H | 0 | S | |
| 1.012 | H | 4-$CH_2Br$ | H | 0 | S | |
| 1.013 | F | 4-$CH_2Br$ | H | 0 | S | |
| 1.014 | H | 4-$CH_2CN$ | H | 0 | S | 82- 84 |
| 1.015 | F | 4-$CH_2CN$ | H | 0 | S | |
| 1.016 | H | 4-$CH_2OH$ | H | 0 | S | 88- 90 |

Tabelle 1 (Fortsetzung)

| Nr. | $R^1$ | $R^3$ | $R^4$ | n | X | Fp.[°C] |
|---|---|---|---|---|---|---|
| 1.017 | F | $4-CH_2OH$ | H | 0 | S | |
| 1.018 | H | $4-CH_2SH$ | H | 0 | S | 96- 97 |
| 1.019 | F | $4-CH_2SH$ | H | 0 | S | |
| 1.020 | H | $4-CH_2COOCH_3$ | H | 0 | S | |
| 1.021 | F | $4-CH_2COOCH_3$ | H | 0 | S | |
| 1.022 | H | $4-CH_2CO_2CH_2CH_3$ | H | 0 | S | |
| 1.023 | F | $4-CH_2CO_2CH_2CH_3$ | H | 0 | S | |
| 1.024 | H | $4-CH_2OCH_3$ | H | 0 | S | |
| 1.025 | F | $4-CH_2OCH_3$ | H | 0 | S | |
| 1.026 | H | $4-CH_2OCH_2CH_3$ | H | 0 | S | |
| 1.027 | F | $4-CH_2OCH_2CH_3$ | H | 0 | S | |
| 1.028 | H | $4-CH_2SCH_3$ | H | 0 | S | Öl |
| 1.029 | F | $4-CH_2SCH_3$ | H | 0 | S | |
| 1.030 | H | $4-CH_2CH_2SCH_3$ | H | 0 | S | |
| 1.031 | F | $4-CH_2CH_2SCH_3$ | H | 0 | S | |
| 1.032 | H | $4-CH_2OCH_2CH=CH_2$ | H | 0 | S | |
| 1.033 | F | $4-CH_2OCH_2CH=CH_2$ | H | 0 | S | |
| 1.034 | H | $4-CH_2OCH_2C \equiv CH$ | H | 0 | S | |
| 1.035 | F | $4-CH_2OCH_2C \equiv CH$ | H | 0 | S | |
| 1.036 | H | $4-CH_2OCOCH_3$ | H | 0 | S | 80- 81 |
| 1.037 | F | $4-CH_2OCOCH_3$ | H | 0 | S | |
| 1.038 | H | $4-CH_2OCOCH_2CH_3$ | H | 0 | S | |
| 1.039 | F | $4-CH_2OCOCH_2CH_3$ | H | 0 | S | |
| 1.040 | H | $4-CO_2CH_3$ | H | 0 | S | |

Tabelle 1 (Fortsetzung)

| Nr. | $R^1$ | $R^3$ | $R^4$ | n | X | Fp.[°C] |
|---|---|---|---|---|---|---|
| 1.041 | F | $4-CO_2CH_3$ | H | 0 | S | |
| 1.042 | H | $4-CO_2CH_2CH_3$ | H | 0 | S | |
| 1.043 | F | $4-CO_2CH_2CH_3$ | H | 0 | S | |
| 1.044 | H | 4-COOH | H | 0 | S | |
| 1.045 | F | 4-COOH | H | 0 | S | |
| 1.046 | H | $4-CH_2SCH_2CO_2CH_3$ | H | 0 | S | Öl |
| 1.047 | F | $4-CH_2SCH_2CO_2CH_3$ | H | 0 | S | |
| 1.048 | H | $4-CH_2SCH_2CO_2CH_2CH_3$ | H | 0 | S | |
| 1.049 | F | $4-CH_2SCH_2CO_2CH_2CH_3$ | H | 0 | S | |
| 1.050 | H | $4-CH_2OCH_2CO_2CH_3$ | H | 0 | S | |
| 1.051 | F | $4-CH_2OCH_2CO_2CH_3$ | H | 0 | S | |
| 1.052 | H | $4-CH_2OCH_2CO_2CH_2CH_3$ | H | 0 | S | |
| 1.053 | F | $4-CH_2OCH_2CO_2CH_2CH_3$ | H | 0 | S | |
| 1.054 | H | $4-CH_3$ | $5-CH_3$ | 0 | S | |
| 1.055 | F | $4-CH_3$ | $5-CH_3$ | 0 | S | |
| 1.056 | H | $4-CH_2SCH_2CH=CH_2$ | H | 0 | S | Öl |
| 1.057 | F | $4-CH_2SCH_2CH=CH_2$ | H | 0 | S | |
| 1.058 | H | $4-CH_2SCH_2C\equiv CH$ | H | 0 | S | Öl |
| 1.059 | F | $4-CH_2SCH_2C\equiv CH$ | H | 0 | S | |
| 1.060 | H | $4-CH_2SCH(CH_3)CO_2CH_3$ | H | 0 | S | Öl |
| 1.061 | F | $4-CH_2SCH(CH_3)CO_2CH_3$ | H | 0 | S | |

Tabelle 2

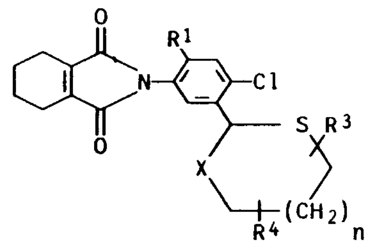

| Nr. | $R^1$ | $R^3$ | $R^4$ | n | X | Fp. [°C] |
|---|---|---|---|---|---|---|
| 2.001 | H | H | H | 0 | 0 | 141-142 |
| 2.002 | F | H | H | 0 | 0 | |
| 2.003 | H | $4-CH_3$ | H | 0 | 0 | öl |
| 2.004 | F | $4-CH_3$ | H | 0 | 0 | öl |
| 2.005 | H | $4-CH_2CH_3$ | H | 0 | 0 | |
| 2.006 | F | $4-CH_2CH_3$ | H | 0 | 0 | |
| 2.007 | H | $4-(CH_2)_2CH_3$ | H | 0 | 0 | |
| 2.008 | F | $4-(CH_2)_2CH_3$ | H | 0 | 0 | |
| 2.009 | H | $4-CH(CH_3)_2$ | H | 0 | 0 | |
| 2.010 | F | $4-CH(CH_3)_2$ | H | 0 | 0 | |
| 2.011 | H | $4-CH_2Cl$ | H | 0 | 0 | |
| 2.012 | F | $4-CH_2Cl$ | H | 0 | 0 | |
| 2.013 | H | $4-CH_2Br$ | H | 0 | 0 | |
| 2.014 | F | $4-CH_2Br$ | H | 0 | 0 | |
| 2.015 | H | $4-CH_2CN$ | H | 0 | 0 | |
| 2.016 | F | $4-CH_2CN$ | H | 0 | 0 | |
| 2.017 | H | $4-CH_2OH$ | H | 0 | 0 | 91-93 |

Tabelle 2 (Fortsetzung)

| Nr. | $R^1$ | $R^3$ | $R^4$ | n | X | Fp.[°C] |
|---|---|---|---|---|---|---|
| 2.018 | F | $4-CH_2OH$ | H | 0 | 0 | |
| 2.019 | H | $4-CH_2SH$ | H | 0 | 0 | |
| 2.020 | F | $4-CH_2SH$ | H | 0 | 0 | |
| 2.021 | H | $4-CH_2CO_2CH_3$ | H | 0 | 0 | |
| 2.022 | F | $4-CH_2CO_2CH_3$ | H | 0 | 0 | |
| 2.023 | H | $4-CH_2CO_2CH_2CH_3$ | H | 0 | 0 | |
| 2.024 | F | $4-CH_2CO_2CH_2CH_3$ | H | 0 | 0 | |
| 2.025 | H | $4-CH_2OCH_3$ | H | 0 | 0 | |
| 2.026 | F | $4-CH_2OCH_3$ | H | 0 | 0 | |
| 2.027 | H | $4-CH_2OCH_2CH_3$ | H | 0 | 0 | |
| 2.028 | F | $4-CH_2OCH_2CH_3$ | H | 0 | 0 | |
| 2.029 | H | $4-CH_2SCH_3$ | H | 0 | 0 | |
| 2.030 | F | $4-CH_2SCH_3$ | H | 0 | 0 | |
| 2.031 | H | $4-CH_2CH_2SCH_3$ | H | 0 | 0 | |
| 2.032 | F | $4-CH_2CH_2SCH_3$ | H | 0 | 0 | |
| 2.033 | H | $4-CH_2OCH_2CH=CH_2$ | H | 0 | 0 | |
| 2.034 | F | $4-CH_2OCH_2CH=CH_2$ | H | 0 | 0 | |
| 2.035 | H | $4-CH_2OCH_2C\equiv CH$ | H | 0 | 0 | |
| 2.036 | F | $4-CH_2OCH_2C\equiv CH$ | H | 0 | 0 | |
| 2.037 | H | $4-CH_2OCOCH_3$ | H | 0 | 0 | |
| 2.038 | F | $4-CH_2OCOCH_3$ | H | 0 | 0 | |
| 2.039 | H | $4-CH_2OCOCH_2CH_3$ | H | 0 | 0 | |
| 2.040 | F | $4-CH_2OCOCH_2CH_3$ | H | 0 | 0 | |
| 2.041 | H | $4-CO_2CH_3$ | H | 0 | 0 | |

Tabelle 2 (Fortsetzung)

| Nr. | $R^1$ | $R^3$ | $R^4$ | n | X | Fp. [°C] |
|---|---|---|---|---|---|---|
| 2.042 | F | $4-CO_2CH_3$ | H | 0 | 0 | |
| 2.043 | H | $4-CO_2CH_2CH_3$ | H | 0 | 0 | |
| 2.044 | F | $4-CO_2CH_2CH_3$ | H | 0 | 0 | |
| 2.045 | H | 4-COOH | H | 0 | 0 | |
| 2.046 | F | 4-COOH | H | 0 | 0 | |
| 2.047 | H | $4-CH_2SCH_2CO_2CH_3$ | H | 0 | 0 | |
| 2.048 | F | $4-CH_2SCH_2CO_2CH_3$ | H | 0 | 0 | |
| 2.049 | H | $4-CH_2SCH_2CO_2CH_2CH_3$ | H | 0 | 0 | |
| 2.050 | F | $4-CH_2SCH_2CO_2CH_2CH_3$ | H | 0 | 0 | |
| 2.051 | H | $4-CH_2OCH_2CO_2CH_3$ | H | 0 | 0 | |
| 2.052 | F | $4-CH_2OCH_2CO_2CH_3$ | H | 0 | 0 | |
| 2.053 | H | $4-CH_2OCH_2CO_2CH_2CH_3$ | H | 0 | 0 | |
| 2.054 | F | $4-CH_2OCH_2CO_2CH_2CH_3$ | H | 0 | 0 | |
| 2.055 | H | $4-CH_3$ | $5-CH_3$ | 0 | 0 | |
| 2.056 | F | $4-CH_3$ | $5-CH_3$ | 0 | 0 | |
| 2.057 | H | H | $5-CH_3$ | 0 | 0 | |
| 2.058 | F | H | $5-CH_3$ | 0 | 0 | |
| 2.059 | H | H | $5-CH_2CH_3$ | 0 | 0 | |
| 2.060 | F | H | $5-CH_2CH_3$ | 0 | 0 | |
| 2.061 | H | H | $5-(CH_2)_2CH_3$ | 0 | 0 | |
| 2.062 | F | H | $5-(CH_2)_2CH_3$ | 0 | 0 | |
| 2.063 | H | H | $5-CH(CH_3)_2$ | 0 | 0 | |
| 2.064 | F | H | $5-CH(CH_3)_2$ | 0 | 0 | |
| 2.065 | H | $5-CH_2Cl$ | H | 0 | 0 | |
| 2.066 | F | $5-CH_2Cl$ | H | 0 | 0 | |

Tabelle 2 (Fortsetzung)

| Nr. | $R^1$ | $R^3$ | $R^4$ | n | X | Fp. [°C] |
|---|---|---|---|---|---|---|
| 2.067 | H | 5-$CH_2Br$ | H | 0 | 0 | |
| 2.068 | F | 5-$CH_2Br$ | H | 0 | 0 | |
| 2.069 | H | 5-$CH_2CN$ | H | 0 | 0 | |
| 2.070 | F | 5-$CH_2CN$ | H | 0 | 0 | |
| 2.071 | H | 5-$CH_2OH$ | H | 0 | 0 | |
| 2.072 | F | 5-$CH_2OH$ | H | 0 | 0 | |
| 2.073 | H | 5-$CH_2SH$ | H | 0 | 0 | |
| 2.074 | F | 5-$CH_2SH$ | H | 0 | 0 | |
| 2.075 | H | 5-$CH_2CO_2CH_3$ | H | 0 | 0 | |
| 2.076 | F | 5-$CH_2CO_2CH_3$ | H | 0 | 0 | |
| 2.077 | H | 5-$CH_2CO_2CH_2CH_3$ | H | 0 | 0 | |
| 2.078 | F | 5-$CH_2CO_2CH_2CH_3$ | H | 0 | 0 | |
| 2.079 | H | 5-$CH_2OCH_3$ | H | 0 | 0 | |
| 2.080 | F | 5-$CH_2OCH_3$ | H | 0 | 0 | |
| 2.081 | H | 5-$CH_2OCH_2CH_3$ | H | 0 | 0 | |
| 2.282 | F | 5-$CH_2OCH_2CH_3$ | H | 0 | 0 | |
| 2.083 | H | 5-$CH_2SCH_3$ | H | 0 | 0 | |
| 2.084 | F | 5-$CH_2SCH_3$ | H | 0 | 0 | |
| 2.085 | H | 5-$CH_2OCH_2CH{=}CH_2$ | H | 0 | 0 | |
| 2.086 | F | 5-$CH_2OCH_2CH{=}CH_2$ | H | 0 | 0 | |
| 2.087 | H | 5-$CH_2OCH_2C{\equiv}CH$ | H | 0 | 0 | |
| 2.088 | F | 5-$CH_2OCH_2C{\equiv}CH$ | H | 0 | 0 | |
| 2.089 | H | 5-$CH_2OCOCH_3$ | H | 0 | 0 | |
| 2.090 | F | 5-$CH_2OCOCH_3$ | H | 0 | 0 | |
| 2.091 | H | 5-$CH_2OCOCH_2CH_3$ | H | 0 | 0 | |

Tabelle 2 (Fortsetzung)

| Nr. | $R^1$ | $R^3$ | $R^4$ | n | X | Fp. [°C] |
|---|---|---|---|---|---|---|
| 2.092 | F | $5-CH_2OCOCH_2CH_3$ | H | 0 | 0 | |
| 2.093 | H | $5-CO_2CH_3$ | H | 0 | 0 | |
| 2.094 | F | $5-CO_2CH_3$ | H | 0 | 0 | |
| 2.095 | H | $5-CO_2CH_2CH_3$ | H | 0 | 0 | |
| 2.096 | F | $5-CO_2CH_2CH_3$ | H | 0 | 0 | |
| 2.097 | H | 5-COOH | H | 0 | 0 | |
| 2.098 | F | 5-COOH | H | 0 | 0 | |
| 2.099 | H | $5-CH_2SCH_2CO_2CH_3$ | H | 0 | 0 | |
| 2.100 | F | $5-CH_2SCH_2CO_2CH_3$ | H | 0 | 0 | |
| 2.101 | H | $5-CH_2SCH_2CO_2CH_2CH_3$ | H | 0 | 0 | |
| 2.102 | F | $5-CH_2SCH_2CO_2CH_2CH_3$ | H | 0 | 0 | |
| 2.103 | H | $5-CH_2OCH_2CO_2CH_3$ | H | 0 | 0 | |
| 2.104 | F | $5-CH_2OCH_2CO_2CH_3$ | H | 0 | 0 | |
| 2.105 | H | $5-CH_2OCH_2CO_2CH_3$ | H | 0 | 0 | |
| 2.106 | F | $5-CH_2OCH_2CO_2CH_3$ | H | 0 | 0 | |

Tabelle 3

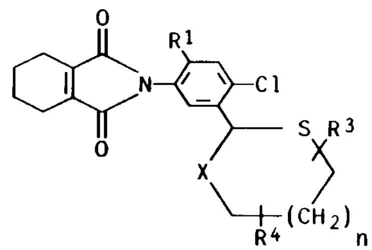

| Nr. | $R^1$ | $R^3$ | $R^4$ | n | X | Fp. [°C] |
|---|---|---|---|---|---|---|
| 3.001 | H | H | H | 1 | S | 198-200 |
| 3.002 | F | H | H | 1 | S | |
| 3.003 | H | $4-CH_3$ | H | 1 | S | 158-159 |
| 3.004 | F | $4-CH_3$ | H | 1 | S | |
| 3.005 | H | $4-CH_2CH_3$ | H | 1 | S | |
| 3.006 | F | $4-CH_2CH_3$ | H | 1 | S | |
| 3.007 | H | $4-(CH_2)_2CH_3$ | H | 1 | S | |
| 3.008 | F | $4-(CH_2)_2CH_3$ | H | 1 | S | |
| 3.009 | H | $4-CH(CH_3)_2$ | H | 1 | S | |
| 3.010 | F | $4-CH(CH_3)_2$ | H | 1 | S | |
| 3.011 | H | $4-CH_2Cl$ | H | 1 | S | |
| 3.012 | F | $4-CH_2Cl$ | H | 1 | S | |
| 3.013 | H | $4-CH_2Br$ | H | 1 | S | |
| 3.014 | F | $4-CH_2Br$ | H | 1 | S | |
| 3.015 | H | $4-CH_2CN$ | H | 1 | S | |
| 3.016 | F | $4-CH_2CN$ | H | 1 | S | |
| 3.017 | H | $4-CH_2OH$ | H | 1 | S | |
| 3.018 | F | $4-CH_2OH$ | H | 1 | S | |
| 3.019 | H | $4-CH_2SH$ | H | 1 | S | |

Tabelle 3 (Fortsetzung)

| Nr. | $R^1$ | $R^3$ | $R^4$ | n | X | Fp. [°C] |
|---|---|---|---|---|---|---|
| 3.020 | F | $4-CH_2SH$ | H | 1 | S | |
| 3.021 | H | $4-CH_2CO_2CH_3$ | H | 1 | S | |
| 3.022 | F | $4-CH_2CO_2CH_3$ | H | 1 | S | |
| 3.023 | H | $4-CH_2CO_2CH_2CH_3$ | H | 1 | S | |
| 3.024 | F | $4-CH_2CO_2CH_2CH_3$ | H | 1 | S | |
| 3.025 | H | $4-CH_2OCH_3$ | H | 1 | S | |
| 3.026 | F | $4-CH_2OCH_3$ | H | 1 | S | |
| 3.027 | H | $4-CH_2OCH_2CH_3$ | H | 1 | S | |
| 3.028 | F | $4-CH_2OCH_2CH_3$ | H | 1 | S | |
| 3.029 | H | $4-CH_2SCH_3$ | H | 1 | S | |
| 3.030 | F | $4-CH_2SCH_3$ | H | 1 | S | |
| 3.031 | H | $4-CH_2CH_2SCH_3$ | H | 1 | S | |
| 3.032 | F | $4-CH_2CH_2SCH_3$ | H | 1 | S | |
| 3.033 | H | $4-CH_2OCH_2CH=CH_2$ | H | 1 | S | |
| 3.034 | F | $4-CH_2OCH_2CH=CH_2$ | H | 1 | S | |
| 3.035 | H | $4-CH_2OCH_2C \equiv CH$ | H | 1 | S | |
| 3.036 | F | $4-CH_2OCH_2C \equiv CH$ | H | 1 | S | |
| 3.037 | H | $4-CH_2OCOCH_3$ | H | 1 | S | |
| 3.038 | F | $4-CH_2OCOCH_3$ | H | 1 | S | |
| 3.039 | H | $4-CH_2OCOCH_2CH_3$ | H | 1 | S | |
| 3.040 | F | $4-CH_2OCOCH_2CH_3$ | H | 1 | S | |
| 3.041 | H | $4-CO_2CH_3$ | H | 1 | S | |
| 3.042 | F | $4-CO_2CH_3$ | H | 1 | S | |
| 3.043 | H | $4-CO_2CH_2CH_3$ | H | 1 | S | |
| 3.044 | F | $4-CO_2CH_2CH_3$ | H | 1 | S | |

Tabelle 3 (Fortsetzung)

| Nr. | $R^1$ | $R^3$ | $R^4$ | n | X | Fp. [°C] |
|---|---|---|---|---|---|---|
| 3.045 | H | 4-COOH | H | 1 | S | |
| 3.046 | F | 4-COOH | H | 1 | S | |
| 3.047 | H | $4-CH_2SCH_2CO_2CH_3$ | H | 1 | S | |
| 3.048 | F | $4-CH_2SCH_2CO_2CH_3$ | H | 1 | S | |
| 3.049 | H | $4-CH_2SCH_2CO_2CH_2CH_3$ | H | 1 | S | |
| 3.050 | F | $4-CH_2SCH_2CO_2CH_2CH_3$ | H | 1 | S | |
| 3.051 | H | $4-CH_2OCH_2CO_2CH_3$ | H | 1 | S | |
| 3.052 | F | $4-CH_2OCH_2CO_2CH_3$ | H | 1 | S | |
| 3.053 | H | $4-CH_2OCH_2CO_2CH_2CH_3$ | H | 1 | S | |
| 3.054 | F | $4-CH_2OCH_2CO_2CH_2CH_3$ | H | 1 | S | |
| 3.055 | H | $4-CH_3$ | $6-CH_3$ | 1 | S | |
| 3.056 | F | $4-CH_3$ | $6-CH_3$ | 1 | S | |
| 3.057 | H | $5-CH_3$ | $5-CH_3$ | 1 | S | |
| 3.058 | F | $5-CH_3$ | $5-CH_3$ | 1 | S | |

Tabelle 4

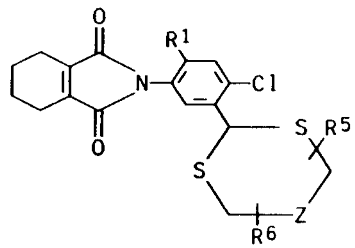

| Nr. | $R^1$ | $R^5$ | $R^6$ | Z | Fp. [°C] |
|---|---|---|---|---|---|
| 4.001 | H | H | H | -CH=CH- | |
| 4.002 | F | H | H | -CH=CH- | |
| 4.003 | H | $4-CH_3$ | H | -CH=CH- | |
| 4.004 | F | $4-CH_3$ | H | -CH=CH- | |
| 4.005 | H | $4-CH_2CH_3$ | H | -CH=CH- | |
| 4.006 | F | $4-CH_2CH_3$ | H | -CH=CH- | |
| 4.007 | H | $4-CH_2CH_2CH_3$ | H | -CH=CH- | |
| 4.008 | F | $4-CH_2CH_2CH_3$ | H | -CH=CH- | |
| 4.009 | H | $4-CH(CH_3)_2$ | H | -CH=CH- | |
| 4.010 | F | $4-CH(CH_3)_2$ | H | -CH=CH- | |
| 4.011 | H | $4-CH_3$ | $7-CH_3$ | -CH=CH- | |
| 4.012 | F | $4-CH_3$ | $7-CH_3$ | -CH=CH- | |
| 4.013 | H | H | H | $-CH_2OCH_2-$ | 134-135 |
| 4.014 | F | H | H | $-CH_2OCH_2-$ | |
| 4.015 | H | $4-CH_3$ | H | $-CH_2OCH_2-$ | |
| 4.016 | F | $4-CH_3$ | H | $-CH_2OCH_2-$ | |
| 4.017 | H | $4-CH_2CH_3$ | H | $-CH_2OCH_2-$ | |

Tabelle 4 (Fortsetzung)

| Nr. | $R^1$ | $R^5$ | $R^6$ | Z | Fp. [°C] |
|---|---|---|---|---|---|
| 4.018 | F | 4-$CH_2CH_3$ | H | -$CH_2OCH_2$- | |
| 4.019 | H | 4-$CH_2CH_2CH_3$ | H | -$CH_2OCH_2$- | |
| 4.020 | F | 4-$CH_2CH_2CH_3$ | H | -$CH_2OCH_2$- | |
| 4.021 | H | 4-$CH(CH_3)_2$ | H | -$CH_2OCH_2$- | |
| 4.022 | F | 4-$CH(CH_3)_2$ | H | -$CH_2OCH_2$- | |
| 4.023 | H | 4-$CH_3$ | 8-$CH_3$ | -$CH_2OCH_2$- | |
| 4.024 | F | 4-$CH_3$ | 8-$CH_3$ | -$CH_2OCH_2$- | |
| 4.025 | H | H | H | -$CH_2SCH_2$- | |
| 4.026 | F | H | H | -$CH_2SCH_2$- | |
| 4.027 | H | 4-$CH_3$ | H | -$CH_2SCH_2$- | |
| 4.028 | F | 4-$CH_3$ | H | -$CH_2SCH_2$- | |
| 4.029 | H | 4-$CH_2CH_3$ | H | -$CH_2SCH_2$- | |
| 4.030 | F | 4-$CH_2CH_3$ | H | -$CH_2SCH_2$- | |
| 4.031 | H | 4-$CH_2CH_2CH_3$ | H | -$CH_2SCH_2$- | |
| 4.032 | F | 4-$CH_2CH_2CH_3$ | H | -$CH_2SCH_2$- | |
| 4.033 | H | 4-$CH(CH_3)_2$ | H | -$CH_2SCH_2$- | |
| 4.034 | F | 4-$CH(CH_3)_2$ | H | -$CH_2SCH_2$- | |
| 4.035 | H | 4-$CH_3$ | 8-$CH_3$ | -$CH_2SCH_2$- | |
| 4.036 | F | 4-$CH_3$ | 8-$CH_3$ | -$CH_2SCH_2$- | |

Tabelle 5

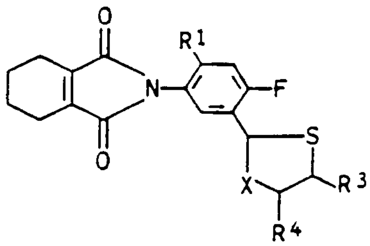

| Nr. | R1 | R3 | R4 | X | Fp. [°C] |
|---|---|---|---|---|---|
| 5.001 | H | $CH_3$ | H | S | |
| 5.002 | F | $CH_3$ | H | S | 110-112 |
| 5.003 | H | H | H | O | |
| 5.004 | F | H | H | O | |
| 5.005 | H | H | $CH_3$ | O | |
| 5.006 | F | H | $CH_3$ | O | Öl |

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadien 0,005 bis 3,0 vorzugsweise 0,01 bis 0,5 kg/ha.

Die herbizide Wirkung der Tetrahydroisoindoldionderivate der Formel I auf das Wachstum der Testpflanzen wird durch folgende Gewächshausversuche gezeigt.

Als Kulturgefäße dienen Plastikblumentöpfe mit 300 $cm^3$ Inhalt und lehmigem Sand mit etwa 3,0 % Humus als Substrat. Die Samen der Testpflanzen werden nach Arten getrennt eingesät.

Zum Zweck der Nachauflaufbehandlung werden entweder direkt gesäte oder in den gleichen Gefäßen aufgewachsene Pflanzen ausgewählt oder sie werden erst als Keimpflanzen getrennt angezogen und einige Tage vor der Behandlung in die Versuchsgefäße verpflanzt.

Je nach Wuchsform werden die Testpflanzen bei einer Wuchshöhe von 3 bis 15 cm dann mit den in Wasser als Verteilungsmittel suspendierten oder emulgierten Wirkstoffen, die durch fein verteilende Düsen gespritzt werden, behandelt. Die Aufwandmenge für die Nachauflaufbehandlung beträgt 0,015 bzw. 0,03 kg/ha a.S.

Die Versuchsgefäße werden im Gewächshaus aufgestellt, wobei für wärmeliebende Arten wärmere Bereiche (20 bis 36 °C) und für solche gemäßigter Klimate 10 bis 20 °C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet.

Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen gehören den folgenden Arten an:

| CODE | Lateinischer Name | Deutscher Name | Englischer Name |
|---|---|---|---|
| ABUTH | Abutilon theophrasti | Chinesischer Hanf | velvet leaf |
| CHYCO | Crysanthemum corinarium | Kronenwucherblume | crown daisy |
| AMARE | Amaranthus spp. | Fuchsschwanzarten | pigweed |
| DEDTO | Desmodium tortuosum | - | Florida beggarweed |
| GALAP | Galium aparine | Klettenlabkraut | catchweed bedstraw |
| IPOSS | Ipomoea spp. | Prunkwindearten | morningglory |
| LAMAM | Lamium amplexicaule | stengelumfassende Taubnessel | henbit |
| MERAN | Mercurialis annua | Einjähriges Bingelkraut | annual mercury |
| SOLNI | Solanum nigrum | Schwarzer Nachtschatten | black nightshade |
| STEME | Stellaria media | Vogelsternmiere | chickweed |
| TRZAS | Triticum aestivum | Sommerweizen | wheat |
| ZEAMX | Zea mays | Mais | Indian corn |

In Anbetracht der Vielseitigkeit der Applikationmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgenden Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napobrassica | Kohlrübe |
| Brassica napus var. rapa | Weiße Rübe |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (C.canephora, Coffea liberica) | Kaffee |
| Cucumis melo | Melone |
| Cynodon dactylon | Bermudagras |
| Elaeis guineensis | Ölpalme |

| Botanischer Name | Deutscher Name |
|---|---|
| Fragaria vesca | Erdbeere |
| Glycine max | Sojabohne |
| Gossypium hirsutum (G. arboreum, Gossypium herbaceum, G. vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |

| Botanischer Name | Deutscher Name |
|---|---|
| Solanum tuberosum | Kartoffel |
| Sorghum bicolor (S. vulgare) | Mohrenhirse |
| Sorghum dochna | Zuckerhirse |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die N-Phenyltetrahydrophthalimide der Formel I sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate (Hetero)-aryloxy-phenoxypropionsäuren sowie deren Salze, Ester und Amide und andere in Betracht.

Außerdem kann es von Nutzen sein, die N-Phenyltetrahydrophthalimide der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Tabelle 6

Herbizide Wirkung und Verträglichkeit für eine Beispielkultur bei Nachauflaufanwendung von Bsp. 1.001 mit 0,03 kg/ha im Gewächshaus

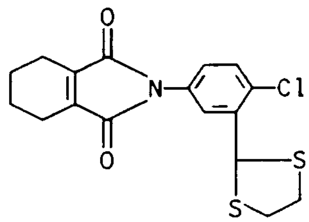

Bsp. 1.001

| Testpflanzen | Schädigung (%) |
|---|---|
| TRZAS | 0 |
| AMARE | 100 |
| GALAP | 100 |
| IPOSS | 100 |
| MERAN | 100 |

Tabelle 7

Herbizide Wirkung bei Nachauflaufanwendung von Bsp. 1.002 mit 0,03 kg/ha im Gewächshaus

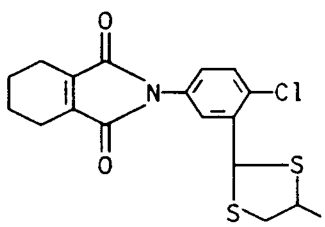

Bsp. 1.002

| Testpflanzen | Schädigung (%) |
|---|---|
| ABUTH | 100 |
| AMARE | 100 |
| DEDTO | 100 |
| GALAP | 100 |
| IPOSS | 100 |
| MERAN | 100 |
| SOLNI | 100 |
| STEME | 100 |

Tabelle 8

**Bekämpfung unerwünschter breitblättriger Pflanzen und** Verträglichkeit für eine Beispielkultur bei Nachauflaufapplikation von 0,015 kg/ha a.S. im Gewächshaus

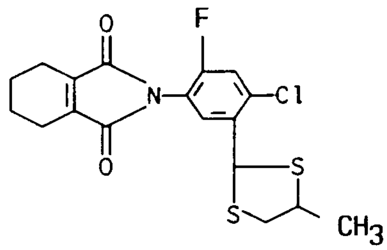

Bsp.-Nr. 1.002

| ZEAMX | CHYCO | GALAP | LAMAM | SOLNI | STEME |
|---|---|---|---|---|---|
| 15 | 100 | 100 | 100 | 100 | 100 |

**Patentansprüche**

**Patentansprüche für folgende Vertragsstaaten : BE, CH, DE, FR, GB, IT, LI, NL, SE**

**1.** N-Phenyltetrahydrophthalimid der allgemeinen Formel I

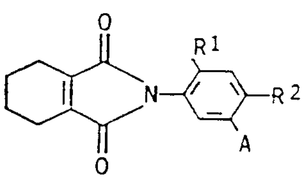

I

in der die Substituenten die folgende Bedeutung haben:

$R^1$ Wasserstoff oder Halogen;

$R^2$ Halogen;

A einen Substituenten der Formel

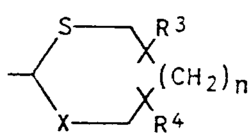

oder

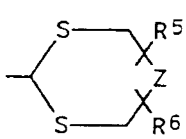

wobei die Variablen die folgende Bedeutung haben:

X Sauerstoff oder Schwefel;

n 0 oder 1;

$R^3$ Wasserstoff, die Hydroxylgruppe, die Carboxylgruppe, eine $C_1$-$C_6$-Alkoxycarbonylgruppe, eine $C_1$-$C_3$-Alkylgruppe, die durch Halogen, Cyano, Hydroxy, Mercapto, $C_1$-$C_6$-Alkoxycarbonyl, $C_1$-$C_4$-Alkoxy, $C_1$-$C_4$-Alkylthio, $C_3$-$C_6$-Alkenyloxy, $C_3$-$C_6$-Alkenylthio, $C_3$-$C_6$-Alkinyloxy, $C_3$-$C_6$-Alkinylthio oder $C_1$-$C_4$-Acyloxy substituiert sein kann, oder eine im Alkylether- oder Thioetherteil durch $C_1$-$C_6$-Alkoxycarbonyl substituierte $C_1$-$C_4$-Alkoxy-$C_1$-$C_2$-alkyl- oder $C_1$-$C_4$-Alkylthio-$C_1$-$C_2$-alkylgruppe;

$R^4$ Wasserstoff oder $C_1$-$C_3$-Alkyl;

Z Methylenoxymethylen, Methylenthiomethylen oder Ethenylen (-CH = CH-);

$R^5$ Wasserstoff oder $C_1$-$C_3$-Alkyl;

$R^6$ Wasserstoff oder $C_1$-$C_3$-Alkyl;

unabhängig von der jeweiligen sterischen Anordnung,
ausgenommen Verbindungen I, in denen A einen Substituenten der Formel

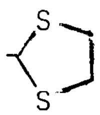

bedeutet, es sei denn $R^1$ steht für Wasserstoff und $R^2$ für Chlor.

**2.** Verfahren zur Herstellung von Verbindungen der Formel I gemäß Anspruch 1, dadurch gekennzeichnet, daß man einen entsprechenden Nitrobenzaldehyd der Formel II

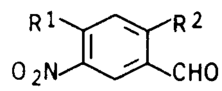

II

mit einem entsprechenden Dithiol oder Mercaptoalkanol der Formel III

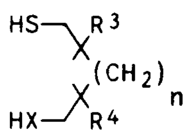

IIIa oder

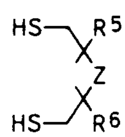

IIIb

umsetzt, das Umsetzungsprodukt zum Anilin reduziert und mit Tetrahydrophthalsäureanhydrid umsetzt.

**3.** Verwendung einer Verbindung der Formel I gemäß Anspruch 1 als Herbizid.

**4.** Herbizid wirkendes Mittel, enthaltend eine Verbindung der Formel I gemäß Anspruch 1 und übliche Hilfsstoffe, Streck- und Verdünnungsmittel.

**5.** Verfahren zur Bekämpfung unerwünschten Pflanzenwuchses, dadurch gekennzeichnet, daß man die Pflanzen und/oder deren Lebensraum mit einer herbizid wirksamen Menge eines N-Phenyltetrahydrophthalimids der Formel 1 gemäß Anspruch 1 behandelt.

**Patentansprüche für folgenden Vertragsstaat : ES**

**1.** Verfahren zur Herstellung von N-Phenyltetrahydrophthalimiden der allgemeinen Formel I

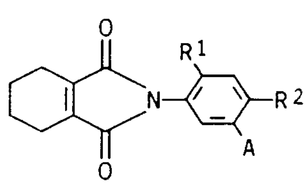

I

in der die Substituenten folgende Bedeutung haben:

$R^1$ Wasserstoff oder Halogen;

$R^2$ Halogen;

A einen Substituenten der Formel

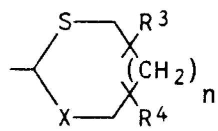

oder

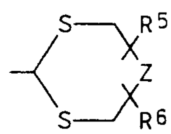

wobei die Variablen die folgende Bedeutung haben:

X Sauerstoff oder Schwefel;

n 0 oder 1;

$R^3$ Wasserstoff, die Hydroxylgruppe, die Carboxylgruppe, eine $C_1$-$C_6$-Alkoxycarbonylgruppe, eine $C_1$-$C_3$-Alkylgruppe, die durch Halogen, Cyano, Hydroxy, Mercapto, $C_1$-$C_6$-Alkoxycarbonyl, $C_1$-$C_4$-Alkoxy, $C_1$-$C_4$-Alkylthio, $C_3$-$C_6$-Alkenyloxy, $C_3$-$C_6$-Alkenylthio, $C_3$-$C_6$-Alkinyloxy, $C_3$-$C_6$-Alkinylthio oder $C_1$-$C_4$-Acyloxy substituiert sein kann, oder eine im Alkylether- oder Thioetherteil durch $C_1$-$C_6$-Alkoxycarbonyl substituierte $C_1$-$C_4$-Alkoxy-$C_1$-$C_2$-alkyl- oder $C_1$-$C_4$-Alkylthio-$C_1$-$C_2$-alkylgruppe;

$R^4$ Wasserstoff oder $C_1$-$C_3$-Alkyl;

Z Methylenoxymethylen, Methylenthiomethylen oder Ethenylen (-CH = CH-);

$R^5$ Wasserstoff oder $C_1$-$C_3$-Alkyl;

$R^6$ Wasserstoff oder $C_1$-$C_3$-Alkyl;

unabhängig von der jeweiligen sterischen Anordnung,

ausgenommen Verbindungen I, in denen A einen Substituenten der Formel

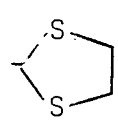

bedeutet, es sei denn $R^1$ steht für Wasserstoff und $R^2$ für Chlor,
dadurch gekennzeichnet, daß man einen entsprechenden Nitrobenzaldehyd der Formel II

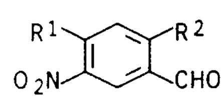

II

mit einem entsprechenden Dithiol oder Mercaptoalkanol der Formel III

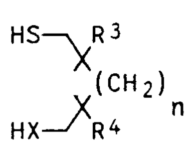

IIIa oder

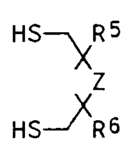

IIIb

umsetzt, das Umsetzungsprodukt zum Anilin reduziert und mit Tetrahydrophthalsäureanhydrid umsetzt.

2. Herbizid wirkendes Mittel, enthaltend eine Verbindung der Formel I gemäß Anspruch 1 und übliche Hilfsstoffe, Streck- und Verdünnungsmittel.

3. Verfahren zur Bekämpfung unerwünschten Pflanzenwuchses, dadurch gekennzeichnet, daß man die Pflanzen und/oder deren Lebensraum mit einer herbizid wirksamen Menge eines N-Phenyltetrahydrophthalimids der Formel 1 gemäß Anspruch 1 behandelt.

**Claims**

**Claims for the following Contracting States : BE, CH, DE, FR, GB, IT, LI, NL, SE**

1. An N-phenyltetrahydrophthalimide of the general formula I

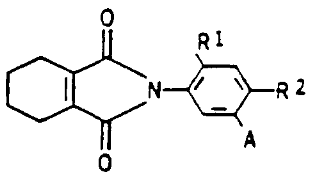

I

where $R^1$ is hydrogen or halogen, $R^2$ is halogen, A is a substituent of the formula

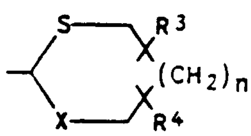

or

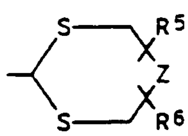

where X is oxygen or sulfur, n is 0 or 1, $R^3$ is hydrogen, hydroxyl, carboxyl, or $C_1$-$C_6$-alkoxycarbonyl, $C_1$-$C_3$-alkyl which may be substituted by halogen, cyano, hydroxyl, mercapto, $C_1$-$C_6$-alkoxycarbonyl, $C_1$-$C_4$-alkoxy, $C_1$-$C_4$-alkylthio, $C_3$-$C_6$-alkenyloxy, $C_3$-$C_6$-alkenylthio, $C_3$-$C_6$-alkynyloxy, $C_3$-$C_6$-alkynylthio, or by $C_1$-$C_4$-acyloxy, or $C_1$-$C_4$-alkoxy-$C_1$-$C_2$-alkyl or $C_1$-$C_4$-alkylthio-$C_1$-$C_2$-alkyl substituted in the alkyl ether or thioether moiety by $C_1$-$C_6$-alkoxycarbonyl, $R^4$ is hydrogen or $C_1$-$C_3$-alkyl, Z is methyleneoxymethylene, methylenethiomethylene or ethenylene (-CH = CH-), $R^5$ is hydrogen or $C_1$-$C_3$-alkyl, and $R^6$ is hydrogen or $C_1$-$C_3$-alkyl, independently of the steric configuration, with the exception of compounds I where A is a substituent of the formula

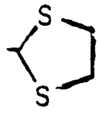

unless $R^1$ is hydrogen and $R^2$ is chlorine.

2. A process for the preparation of a compound of the formula I as claimed in claim 1, wherein a corresponding nitrobenzaldehyde of the formula II

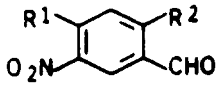

II

is reacted with a corresponding dithiol or mercaptoalkanol of the formula III

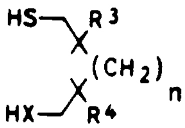 IIIa or 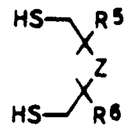 IIIb

and the reaction product is reduced to the aniline and reacted with tetrahydrophthalic anhydride.

**3.** The use of a compound of the formula I as claimed in claim 1 as a herbicide.

**4.** A herbicidal agent containing a compound of the formula I as claimed in claim 1 and conventional auxiliaries, extenders and diluents.

**5.** A method for controlling the growth of undesirable plants, wherein the plants and/or their habitat are treated with a herbicidally effective amount of an N-phenyltetrahydrophthalimide of the formula I as claimed in claim 1.

**Claims for the following Contracting State : ES**

**1.** A process for the preparation of N-phenyltetrahydrophthalimides of the general formula I

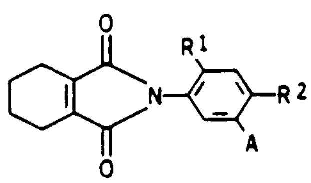

I

where $R^1$ is hydrogen or halogen, $R^2$ is halogen, A is a substituent of the formula

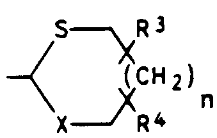

or

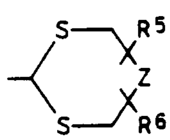

where X is oxygen or sulfur, n is 0 or 1, $R^3$ is hydrogen, hydroxyl, carboxyl, or $C_1$-$C_6$-alkoxycarbonyl, $C_1$-$C_3$-alkyl, which may be substituted by halogen, cyano, hydroxyl, mercapto, $C_1$-$C_6$-alkoxycarbonyl, $C_1$-$C_4$-alkoxy, $C_1$-$C_4$-alkylthio, $C_3$-$C_6$-alkenyloxy, $C_3$-$C_6$-alkenylthio, $C_3$-$C_6$-alkynyloxy, $C_3$-$C_6$-alkynylthio, or by $C_1$-$C_4$-acyloxy, or $C_1$-$C_4$-alkoxy- or $C_1$-$C_4$-alkylthio-$C_1$-$C_2$-alkyl substituted in the alkyl ether or thioether moiety by $C_1$-$C_6$-alkoxycarbonyl, $R^4$ is hydrogen or $C_1$-$C_3$-alkyl, Z is methyleneoxymethylene, methylenethiomethylene or ethenylene (-CH=CH-), $R^5$ is hydrogen or $C_1$-$C_3$-alkyl, and $R^6$ is hydrogen or $C_1$-$C_3$-alkyl, independently of the steric configuration, with the exception of compounds I where A is a substituent of the formula

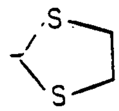

unless $R^1$ is hydrogen and $R^2$ is chlorine, wherein a corresponding nitrobenzaldehyde of the formula II

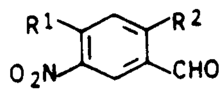

II

is reacted with a corresponding dithiol or mercaptoalkanol of the formula III

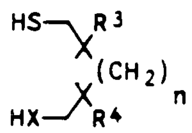

IIIa or

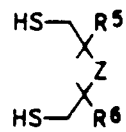

IIIb

and the reaction product is reduced to the aniline and reacted with tetrahydrophthalic anhydride.

**2.** A herbicidal agent containing a compound of the formula I as set forth in claim 1 and conventional auxiliaries, extenders and diluents.

**3.** A method for controlling the growth of undesirable plants, wherein the plants and/or their habitat are treated with a herbicidally effective amount of an N-phenyltetrahydrophthalimide of the formula I as set forth in claim 1.

**Revendications**
**Revendications pour les Etats contractants suivants : BE, CH, DE, FR, GB, IT, LI, NL, SE**

**1.** N-phényltétrahydrophtalimide de formule générale I

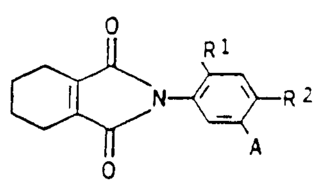

I

dans laquelle les substituants ont les significations suivantes :
$R^1$ hydrogène ou halogène ;
$R^2$ halogène ;
A un substituant de formule

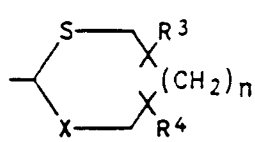

ou

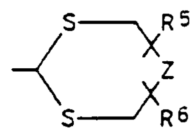

où les variables ont les significations suivantes :
X oxygène ou soufre ;
n 0 ou 1 ;
$R^3$ hydrogène, le groupe hydroxyle, le groupe carboxyle, un groupe alcoxy en C1-C6-carbonyle, un groupe alkyle en C1-C3 qui peut être substitué par halogène, cyano, hydroxy, mercapto, alcoxy en C1-C6-carbonyle, alcoxy en C1-C4, alkyle en C1-C4-thio, alcényl en C3-C6-oxy, alcényl en C3-C6-thio, alcynyl en C3-C6-oxy, alcynyl en C3-C6-thio ou acyle en C1-C4-oxy, ou un groupe alcoxy en C1-C4-alkyle en C1-C2 ou alkylthio en C1-C4-alkyle en C1-C2, substitué dans la partie alkyléther ou thioéther par alcoxy en C1-C6-carbonyle ;
$R^4$ hydrogène ou alkyle en C1-C3 ;
Z méthylènoxyméthylène, méthylènethiométhylène ou éthénylène (-CH = CH-)
$R^5$ hydrogène ou alkyle en C1-C3 ;
$R^6$ hydrogène ou alkyle en C1-C3 ;
indépendamment de l'arrangement stérique respectif, exceptés les composés I dans lesquels A représente un substituant de formule

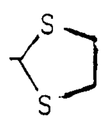

à moins que $R^1$ soit mis pour hydrogène et $R^2$ pour chlore.

2. Procédé de préparation de composés de formule I, selon la revendication 1, caractérisé par le fait que l'on fait réagir un nitrobenzaldéhyde approprié, de formule II

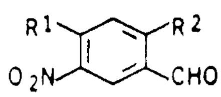

II

avec un dithiol ou un mercaptoalcanol appropriés, de formule III

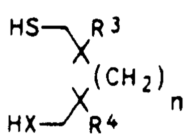

IIIa ou

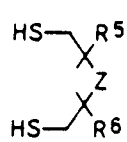

IIIb

on réduit le produit de réaction en aniline et on le met à réagir avec de l'anhydride tétrahydrophtalique.

3. Utilisation d'un composé de formule I, selon la revendication 1, comme herbicide.

4. Agent à action herbicide, contenant un composé de formule I, selon la revendication 1, et des matières auxiliaires, charges et diluants usuels.

5. Procédé pour la lutte contre la croissance de plantes indésirables, caractérisé par le fait que l'on traite les plantes et/ou leur biotope avec une quantité efficace au point de vue herbicide d'un N-phényltétrahydrophtalimide de formule 1, selon la revendication 1.

**Revendications pour l'Etat contractant suivant : ES**

**1.** Procédé de préparation de N-phényltétrahydrophtalimide de formule générale I

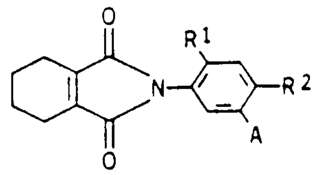

I

dans laquelle les substituants ont les significations suivantes :
$R^1$ hydrogène ou halogène ;
$R^2$ halogène ;
A un substituant de formule

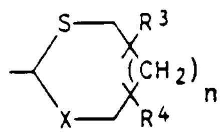

ou

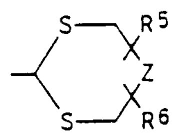

où les variables ont les significations suivantes :
X oxygène ou soufre ;
n 0 ou 1 ;
$R^3$ hydrogène, le groupe hydroxyle, le groupe carboxyle, un groupe alcoxy en C1-C6-carbonyle, un groupe alkyle en C1-C3 qui peut être substitué par halogène, cyano, hydroxy, mercapto, alcoxy en C1-C6-carbonyle, alcoxy en C1-C4, alkyle en C1-C4-thio, alcényl en C3-C6-oxy, alcényl en C3-C6-thio, alcynyl en C3-C6-oxy, alcynyl en C3-C6-thio ou acyle en C1-C4-oxy, ou un groupe alcoxy en C1-C4-alkyle en C1-C2 ou alkylthio en C1-C4-alkyle en C1-C2, substitué dans la partie alkyléther ou thioéther par alcoxy en C1-C6-carbonyle ;
$R^4$ hydrogène ou alkyle en C1-C3 ;
Z méthylènoxyméthylène, méthylènethiométhylène ou éthénylène (-CH = CH-)
$R^5$ hydrogène ou alkyle en C1-C3 ;
$R^6$ hydrogène ou alkyle en C1-C3 ;
indépendamment de l'arrangement stérique respectif, exceptés les composés I dans lesquels A représente un substituant de formule

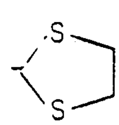

à moins que $R^1$ soit mis pour hydrogène et $R^2$ pour chlore,
caractérisé par le fait que l'on fait réagir un nitrobenzaldéhyde approprié, de formule II

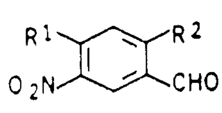

II

avec un dithiol ou un mercaptoalcanol appropriés, de formule III

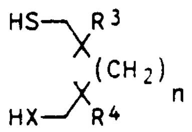 IIIa ou 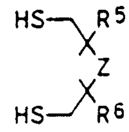 IIIb

on réduit le produit de réaction en aniline et on le met à réagir avec de l'anhydride tétrahydrophtalique.

2. Agent à action herbicide, contenant un composé de formule I, selon la revendication 1, et des matières auxiliaires, charges et diluants usuels.

3. Procédé pour la lutte contre la croissance de plantes indésirables, caractérisé par le fait que l'on traite les plantes et/ou leur biotope avec une quantité efficace au point de vue herbicide d'un N-phényltétrahydrophtalimide de formule 1, selon la revendication 1.